# EUROPÄISCHE PATENTSCHRIFT

(11) **EP 3 404 417 B1**
(45) Veröffentlichungstag und Bekanntmachung des Hinweises auf die Patenterteilung: **13.05.2020**
(21) Anmeldenummer: 18167884.8
(22) Anmeldetag: 18.04.2018
(51) Int. Cl.: G01N 33/68, C12Q 1/04

(54) **PRÄPARATION BIOLOGISCHER ZELLEN AUF MASSENSPEKTROMETRISCHEN PROBENTRÄGERN FÜR EINE DESORBIERENDE IONISIERUNG**
PREPARATION OF BIOLOGICAL CELLS ON MASS SPECTROMETRIC SAMPLE SUPPORTS FOR A DESORBING IONIZATION
PRÉPARATION DE CELLULES BIOLOGIQUES SUR DES SUPPORTS D'ÉCHANTILLONS SPECTROMÉTRIQUES DE MASSE POUR UNE IONISATION PAR DÉSORPTION

(30) Priorität: 15.05.2017 DE 102017110476
(43) Veröffentlichungstag der Anmeldung: 21.11.2018
(73) Patentinhaber: Bruker Daltonik GmbH, 28359 Bremen (DE)
(72) Erfinder: SPARBIER, Katrin, 28357 Bremen (DE); WEGEMANN, Beatrix, 28207 Bremen (DE)
(74) Vertreter: Boßmeyer, Jens

(56) Entgegenhaltungen:
- EP-A1- 2 806 275
- EP-A1- 3 081 652
- DE-A1-102004 019 043
- DE-A1-102010 019 869
- DE-A1-102012 011 647
- DE-A1-102014 000 646
- US-A1- 2005 087 685
- US-A1- 2013 309 712
- AGNÈS FERRONI ET AL: "Real time identification of bacteria and yeast in positive blood 1 culture broths by MALDI-TOF-Mass Spectrometry", JOURNAL OF CLINICAL MICROBIO, 17. März 2010 (2010-03-17), Seite 21pp, XP009131880, ISSN: 0095-1137, DOI: 10.1128/JCM.02485-09 [gefunden am 2010-03-17]
- Bruker: "MALDI Biotyper ? Consumables Added Convenience Enhances Assay Productivity", , 1. Januar 2015 (2015-01-01), XP055377091, Gefunden im Internet: URL:https://www.bruker.com/fileadmin/user_ upload/8-PDF-Docs/Separations_MassSpectrom etry/Literature/Brochures/1838970_MBT_Cons umables_RUO_brochure_08-2015_eBook.pdf [gefunden am 2017-05-30]
- E.A. IDELEVICH ET AL: "Direct blood culturing on solid medium outperforms an automated continuously monitored broth-based blood culture system in terms of time to identification and susceptibility testing", NEW MICROBES AND NEW INFECTIONS, Bd. 10, 1. März 2016 (2016-03-01), Seiten 19-24, XP055374332, ISSN: 2052-2975, DOI: 10.1016/j.nmni.2015.12.004
- Franco Basile: "Rapid Sample Preparation for Microorganism Analysis by Mass Spectrometry" In: "ACS Symposium Series", 1. Januar 2011 (2011-01-01), American Chemical Society/Oxford University Press, US, XP055377083, ISSN: 0097-6156 Bd. 1065, Seiten 5-34, DOI: 10.1021/bk-2011-1065.ch002, * Seite 17; Tabelle 1 *
- LINGJUN LI ET AL: "In Situ Sequencing of Peptides from Biological Tissues and Single Cells Using MALDI-PSD/CID Analysis", ANALYTICAL CHEMISTRY, Bd. 71, Nr. 24, 1. Dezember 1999 (1999-12-01), Seiten 5451-5458, XP055495222, US ISSN: 0003-2700, DOI: 10.1021/ac9907181

## Beschreibung

Die Erfindung bezieht sich auf die Präparation von Proteinen aus biologischen Zellen auf massenspektrometrischen Probenträgern für die massenspektrometrische Analyse im Hinblick auf Zell-Eigenschaften wie taxonomische Einordnung (Identität), Antibiotika-Resistenzen, Verhalten gegenüber Medikamenten oder anderen Wirksubstanzen und andere. Die Zellen können prokaryotische oder eukaryotische Mikroorganismen oder eukaryotische Zellen aus Geweben sein; sie können insbesondere auf Probenträgerplatten in Kulturmedien gezüchtet worden sein, um aus dem Wachstum unter bestimmten Einflüssen, zum Beispiel in Gegenwart bestimmter Substanzen, auf bestimmte Zell-Eigenschaften schließen zu können.

In der Erfindung wird vorgeschlagen, die Zellen auf den massenspektrometrischen Probenträgern nicht durch Zugabe von Matrixlösung für eine spätere Ionisierung durch matrixunterstützte Laserdesorption (MALDI) aufzuschließen, sondern in einem eigenen Behandlungsschritt unter Verwendung von (organischen) Säuren und/oder Lösungsmitteln. Die aus den Zellen freigesetzten schweren Proteine haften dann überraschenderweise auf dem Probenträger, so dass sie unmittelbar dort mit Pufferlösung gewaschen werden können, um Salze und andere lösliche Verunreinigungen zu entfernen, ohne Proteine für die Analyse durch Auswaschen wesentlich zu verlieren.

### Stand der Technik

In der Patentanmeldung PCT/DE2016/100561 (K. Becker und E. Idelevich: "Aufbereitung lebendiger, mikrobieller Proben und Mikroorganismen für anschließende massenspektrometrische Messung und Auswertung") wird detailliert beschrieben, wie Mikroben auf massenspektrometrischen Probenträgerplatten in Nährlösungen inkubiert werden können, beispielsweise mit und ohne Zugabe von Antibiotika, um durch Beobachtung weiteren Wachstums festzustellen, ob eine Resistenz vorliegt oder nicht.

Das in der Schrift beschriebene Verfahren stellt eine neuartige Methode für eine sehr schnelle und einfache MS-basierte Analyse besonderer Eigenschaften von biologischen Zellen dar; beispielsweise bezüglich des Ansprechens von Zellen auf Antibiotika oder Medikamente oder bezüglich weiterer Charakterisierung biologischer Zellen. Die Offenbarung bezieht sich insbesondere auf das Verfahren der Probenverarbeitung und Probenaufbereitung sowie auf Algorithmen der Datenauswertung.

Ein bevorzugter Aspekt der zitierten Schrift betrifft ein Verfahren zur Aufbereitung lebender, mikrobieller Proben für eine anschließende massenspektrometrische Messung, das die folgenden Schritte aufweist: (a) Bereitstellen eines flachen Probenträgers, der mehrere Aufbringungsorte für Proben umfasst (sogenannte "Spots"); (b) Aufbringen wenigstens einer lebenden, mikrobiellen Probe in einem Nährmedium-Tropfen auf wenigstens einen der Probenflecken; (c) Verbringen des Probenträgers in einen Bebrütungsraum mit definierter Atmosphäre für eine vorbestimmte Zeitspanne, um die Mikroorganismen wachsen und sich vermehren zu lassen; (d) Entfernen von Restflüssigkeit des Nährmedium-Tropfens nach der vorbestimmten Zeitspanne, um eine Mikroorganismenablagerung auf dem Probenfleck freizulegen; (e) Präparieren des Probenflecks für eine desorbierende Ionisierung; (f) Überführen des Probenträgers in eine Desorptions-Ionenquelle eines Massenspektrometers, Erzeugen von Ionen aus dem präparierten Probenfleck und Aufnehmen wenigstens eines zugehörigen Massenspektrums; und (g) Abgleichen des aufgenommenen Massenspektrums mit einem Referenzdatensatz, um wenigstens eine Eigenschaft der mikrobiellen Probe zu ermitteln.

Dieses Verfahren kann beispielsweise verwendet werden, um das Wachstum der mikrobiellen Zellen in Anwesenheit verschiedenartiger Antibiotika festzustellen: weiteres Wachstum weist die mikrobiellen Zellen als resistent oder suszeptibel gegenüber dem verwendeten Antibiotikum aus.

Das Verfahren weist gelegentlich Schwierigkeiten auf. Beispielsweise kann das Trennen der Mikroben von Restflüssigkeit mit den genannten Verfahren wie Aufsaugen durch eine Pipette oder durch ein Löschblatt nicht gelingen, wenn die Mikroben an der Oberfläche der Flüssigkeit schwimmen, wie es beispielsweise bei Salmonellen und anderen Flagellaten der Fall ist, denn dann besteht eine hohe Gefahr, die Mikroben mit abzusaugen. Ein Eintrocknen der Nährlösung, um die Zellen auf der Probenträgeroberfläche zu binden und anschließend zu präparieren, hinterlässt aber Salze und andere Bestandteile aus der Nährlösung, die einen schädlichen Einfluss auf die Ionisierung durch matrixunterstützte Laserdesorption ausüben und die Empfindlichkeit für viele Proteine erheblich herabsetzen. Die eingetrockneten Mikrobenproben können oft auch nicht gewaschen werden, da die Mikroben vieler Arten nach dem Eintrocknen der Nährlösung nicht fest genug an der Oberfläche des Probenträgers haften.

Ganz allgemein müssen biologische Zellen für eine Messung ihrer Bestandteile, insbesondere ihrer Proteine, aufgeschlossen werden, um die Proteine für eine spätere Ionisierung freizusetzen. "Aufschließen" bedeutet in diesem Zusammenhang ein Zerstören der Zellwände und ein Auflösen von Proteinkomplexen im Inneren der Zelle, damit die Proteine aus der Zelle austreten können.

Für diesen Aufschluss werden zwei Verfahren empfohlen: (1) ein "externer" Aufschluss durch Zentrifugieren gewaschener Mikroben unter Verwendung von Säuren in einem besonderen Gefäß, weiteres Zentrifugieren und Übertragen des Überstandes mit den Proteinen aus den Zellen auf den massenspektrometrischen Probenträger, und (2) ein Aufschluss der auf den Probenträger aufgebrachten Zellen durch Zugabe einer Lösung der Matrixsubstanz, also einer organischen Säure in einem organischen Lösungsmittel, die die meisten Mikroben aufzuschließen gestattet. Die Matrixlösung bereitet zugleich die Probe für die spätere Ionisierung durch matrixunterstützte Laserdesorption (MALDI) vor. In Verfahren für die Identifizierung von Mikroben liefert der externe Aufschluss einen höheren Prozentsatz an eindeutigen Identifizierungen, dauert aber durch das mehrfache Zentrifugieren deutlich länger und erhöht den Arbeitsaufwand.

In der Arbeit "Evaluation of a Simple Protein Extraction Method for Species Identification of Clinically Relevant Staphylococci by Matrix-Assisted Laser Desorption Ionization-Time of Flight Mass Spectrometry" (N. Matsuda et al., J. Clin. Microbiology, 50, 3862-3866, 2012) wird zusätzlich zu den beiden genannten Verfahren ein Aufschluss der Mikroben auf der Probenträgerplatte mit 70%iger Ameisensäure untersucht, mit ähnlich guten Ergebnissen wie ein externer Aufschluss, aber deutlich geringerem Zeitbedarf und Arbeitsaufwand.

In der Publikation "Evaluation of a Short, On-Plate Formic Acid Extraction Method for Matrix-Assisted Laser Desorption Ionization-Time of Flight Mass Spectrometry-Based Identification of Clinically Relevant Yeast Isolates" (R. L. Gorton et al., J. Clin. Microbiology 52, 1253-1255, 2014) wird ebenfalls ein Aufschluss von Zellen auf der Probenträgerplatte untersucht, allerdings handelt es sich hier um Hefezellen, und der Identifizierungserfolg ist erheblich geringer als bei externem Aufschluss.

In diesen beiden Publikationen wird direkt nach dem Aufschluss auf der Trägerplatte getrocknet und mit Matrixlösung für die Ionisierung durch MALDI vorbereitet. Insbesondere werden Salze oder andere lösliche Verunreinigungen nicht entfernt: die aufgebrachten biologischen Zellen waren offenkundig stets genügend sauber für die MALDI-Präparation.

Es besteht daher nach wie vor ein Bedarf für ein Verfahren, das mit Salzen und anderen löslichen Verunreinigungen behaftete Zellen auf einem massenspektrometrischen Probenträger, beispielsweise nach einem Inkubieren in einem Nährmitteltropfen unmittelbar auf dem Probenträger, so zu präparieren gestattet, dass die Empfindlichkeit der desorbierenden Ionisierung (beispielsweise durch MALDI) nahezu uneingeschränkt erhalten bleibt.

### Kurze Beschreibung der Erfindung

In der Erfindung wird nun vorgeschlagen, die biologischen Zellen auf dem Probenfleck eines massenspektrometrischen Probenträgers nicht durch Zugabe einer Matrixlösung aufzuschließen, sondern in einem eigenen Behandlungsschritt unter Verwendung von matrixfreien (organischen) Säuren und/oder Lösungsmitteln. Die durch diesen Aufschluss freigesetzten Zell-Proteine haften überraschend gut auf der Oberfläche des Probenträgers und können mit Waschpuffer, zum Beispiel reinem Wasser, gewaschen werden, um Salze, Detergenzien, Puffermaterialien und andere lösliche Verunreinigungen, die von vorherigen Bearbeitungsschritten herrühren können, zu entfernen.

Gemäß einem ersten Aspekt betrifft die Erfindung ein Verfahren zur Aufbereitung von Proteinen aus Proben ungereinigter biologischer Zellen (z.B. Mikroorganismen) auf einem Probenträger zur massenspektrometrischen Bestimmung von Zell-Eigenschaften, beispielsweise die taxonomische Einordnung oder das Resistenzverhalten von Mikroorganismen gegenüber einer antimikrobiellen Substanz, aufweisend die Schritte:
- Bereitstellen der biologischen Zellen auf einem Probenfleck des Probenträgers, der eine hydrophile Oberfläche, z.B. eine Platte aus Edelstahl oder eine Platte aus beschichtetem Keramikmaterial, oder eine Platte mit hydrophilen Ankern in hydrophober Umgebung ("AnchorChip™")aufweist,
- Aufschließen der Zellen unter Verwendung von denaturierenden flüchtigen Lösungsmitteln und/oder (organischen) Säuren auf dem Probenfleck derart, dass sich Zell-Proteine aus Komplexen lösen, aus den aufgeschlossenen Zellen austreten und auf dem Probenfleck adhäsiv gebunden werden,
- Waschen der Zell-Proteine auf dem Probenfleck mit (ruhender) Pufferlösung, beispielsweise indem einige Mikroliter einer wässerigen Lösung (insbesondere reines deionisiertes Wasser) auf den Probenfleck aufgebracht werden, dort für eine vorbestimmte Zeitspanne verbleiben und dann abgezogen werden, wobei Salze und andere lösliche Bestandteile in Lösung gehen und mit der Pufferlösung entfernt werden, und
- Präparieren der gewaschenen Zell-Proteine für eine folgende desorbierende Ionisierung, beispielsweise indem Matrixlösung für eine spätere Ionisierung durch matrixunterstützte Laserdesorption auf dem Probenfleck hinzugefügt und auskristallisiert wird.

In verschiedenen Ausführungsformen kann das Bereitstellen der biologischen Zellen die Züchtung der Mikroorganismen in Nährflüssigkeit auf dem Probenfleck des massenspektrometrischen Probenträgers umfassen. Alternativ kann die Züchtung von Mikroorganismen auch in einem externen Züchtungsgefäß mit anschließender Übertragung der (ungereinigten) gezüchteten Mikroorganismen auf den Probenfleck des Probenträgers erfolgen.

Wenn die biologischen Zellen Mikroorganismen aufweisen, lassen sich diese auf verschiedenen Probenflecken des Probenträgers in Nährflüssigkeit mit und ohne Zugabe einer antimikrobiellen Substanz kultivieren, und das Resistenzverhalten gegenüber der antimikrobiellen Substanz kann dann durch weiteres Wachstum in Anwesenheit dieser Substanz bestimmt werden.

In verschiedenen Ausführungsformen kann das Wachstum in Anwesenheit der antimikrobiellen Substanz durch Vergleich mit einer dotiert zugegebenen Referenzsubstanz bestimmt werden; siehe insbesondere EP 2 806 275 B1 und WO 2014/187517 A1.

Vorzugsweise werden die Zell-Eigenschaften, insbesondere von Mikroorganismen, auf Basis von massenspektrometrischen Proteinsignalen im Massenbereich über m/z 3000 bestimmt, insbesondere zwischen etwa m/z 3000 und m/z 15.000.

Eine bevorzugte Variante eines Verfahrens für die Feststellung von Resistenzen gegen Antibiotika/Antimykotika gemäß Prinzipien der Erfindung kann beispielsweise folgende Schritte umfassen:
(1) Zellen in Kulturmedien mit und ohne Antibiotika/Antimykotika auf verschiedene Probenflecken des Probenträgers geben,
(2) Inkubation für eine Spezies- und Antibiotika-abhängige Zeit in einer Kammer, die konstante Bedingungen (insbesondere Feuchtigkeit und Temperatur) schafft,
(3) Proben auf den Probenflecken bei erhöhter Temperatur eintrocknen lassen,
(4) 1 Mikroliter 70%ige Ameisensäure in Wasser zugeben, um die Zellen aufzuschließen und die Proteine herausdiffundieren zu lassen,
(5) Aufgeschlossene Zellen auf den Probenflecken bei erhöhter Temperatur eintrocknen lassen,
(6) 3 Mikroliter H₂O auf die Probenflecken geben, für kurze Zeit (~3 Minuten) einwirken lassen und absaugen (entweder mit einer Pipette oder einer speziellen Vorrichtung zur Entfernung von Flüssigkeiten von den Probenflecken),
(7) Restliche Feuchtigkeit auf den Probenflecken eintrocknen lassen,
(8) Matrixlösung (gegebenenfalls mit einem internen Standard) auf die Probenflecken pipettieren und trocknen lassen,
(9) Messung der Proteinprofile mit einem Massenspektrometer, zum Beispiel einem MALDI-Flugzeitmassenspektrometer.

In Schritt (3) kann die Temperatur beispielsweise zwischen 30° und 60° Celsius, in Grenzfällen noch höher, liegen. In Schritt (4) können auch andere denaturierende flüchtige Lösungsmittel verwendet werden, z.B. 50%iges Aceton in Wasser oder 50 %iges Acetonitril, 2,5 %ige Trifluoressigsäure und 47,5 % Wasser in Wasser.

Das hier vorgeschlagene Verfahren mit Waschprozedur unmittelbar auf dem Probenträger dauert zwar einige Minuten länger als eines ohne, zeigt aber deutliche Vorteile: Die schweren, aus der Zelle freigesetzten Proteine bleiben während des Waschschritts erstaunlicherweise relativ fest auf der Oberfläche des Probenträgers haften, sie sind dadurch weitgehend unlöslich; nur die Salze und andere lösliche Bestandteile, beispielsweise aus dem Kulturmedium, gehen in Lösung und werden mit dem Waschpuffer entfernt. Das funktioniert in überraschender Weise sowohl auf Probenträgerplatten aus poliertem Edelstahl, wie auch auf den bekannten Anker-Targets ("Anchor-Chip™") und auf den neu im Markt befindlichen "Biotargets" aus keramischem Material. Folgte man hingegen der in der Patentanmeldung PCT/DE2016/100561 als bevorzugt dargestellten Vorgehensweise des Absaugens des Kulturüberstands von einem Probenfleck anstatt diesen Eintrocknen zu lassen, so bestände die Gefahr, dass mikrobielle Spezies, die keine oder nur geringe Adhäsion zur Probenträgeroberfläche haben, verloren gehen. Als Beispiel können hier einige Salmonellenarten genannt werden, die als Flagellaten an der Flüssigkeitsoberfläche schwimmen und daher leicht abgesaugt werden könnten.

Vergleichsmessungen zeigen, dass die unter Einsatz des Waschens auf dem Probenträger gewonnenen Spektren eine deutlich verbesserte Qualität gegenüber Spektren von ungewaschenen Proben aufweisen, weil der Anteil an Salzen, die die Ionisierung (insbesondere beim MALDI-Verfahren) stören, durch den Waschschritt stark reduziert wird.

Gemäß einem weiteren Aspekt betrifft die Erfindung insbesondere ein Verfahren zur Aufbereitung von Proteinen aus Proben biologischer Zellen (z.B. Mikroorganismen) zur massenspektrometrischen Bestimmung von Zell-Eigenschaften, beispielsweise die taxonomische Einordnung oder das Resistenzverhalten von Mikroorganismen gegenüber einer antimikrobiellen Substanz, aufweisend die Schritte:
- Kultivieren der biologischen Zellen in Nährflüssigkeit unmittelbar auf einem Probenfleck eines massenspektrometrischen Probenträgers, der eine hydrophile Oberfläche. z.B. eine Platte aus Edelstahl oder eine Platte aus beschichtetem Keramikmaterial, oder eine Platte mit hydrophilen Ankern in hydrophober Umgebung ("AnchorChip™") aufweist,
- Aufschließen der kultivierten Zellen auf dem Probenfleck unter Verwendung von denaturierenden flüchtigen Lösungsmitteln und/oder (organischen) Säuren derart, dass sich Zell-Proteine aus Komplexen lösen, aus den aufgeschlossenen Zellen austreten und auf dem Probenfleck adhäsiv gebunden werden,
- Waschen der Zell-Proteine auf dem Probenfleck mit (ruhender) Pufferlösung, beispielsweise indem einige Mikroliter einer wässerigen Lösung (insbesondere reines deionisiertes Wasser) auf den Probenfleck aufgebracht werden, dort für eine vorbestimmte Zeitspanne verbleiben und dann abgezogen werden, wobei Salze und andere lösliche Bestandteile in Lösung gehen und mit der Pufferlösung entfernt werden, und
- Präparieren der gewaschenen Zell-Proteine auf dem Probenfleck für eine folgende desorbierende Ionisierung, beispielsweise indem Matrixlösung für eine spätere Ionisierung durch matrixunterstützte Laserdesorption auf dem Probenfleck hinzugefügt und auskristallisiert wird.

Als Waschpuffer für die in dieser Offenbarung beschriebenen Verfahren hat sich insbesondere deionisiertes Wasser, rein oder leicht angesäuert, als geeignet erwiesen.

### Kurze Beschreibung der Abbildungen

Abbildung 1 zeigt die Schritte eines Verfahrens, das der Resistenzbestimmung von Mikroorganismen dient.
Abbildung 2 illustriert Massenspektren eines antibiotika-empfindlichen Bakterienstammes (*E. coli*) und eines antibiotika-resistenten Bakterienstammes (*Klebsiella pneumoniae*) nach Züchtung jeweils in Abwesenheit und Gegenwart eines Antibiotikums, die nach Ausführung einer in Abbildung 1 skizzierten Prozedur aufgenommen wurden. Das Spektrum B zeigt allein das Signal einer Referenzsubstanz.
Abbildung 3 gibt ein einfaches Verfahren zur taxonomischen Identifizierung von verunreinigten Mikroorganismen unter Einsatz eines Waschschritts nach Prinzipien der Erfindung wieder.
Abbildung 4 zeigt ein Massenspektrum der reinen Nährflüssigkeit, die wie der erfindungsgemäße Arbeitsablauf unter Verwendung des Waschens der adhäsiv gebundenen Zell-Proteine nach Abbildung 1 behandelt und gewaschen wurde. Obwohl die Nährflüssigkeit Peptide enthält, ist das Massenspektrum sauber und enthält oberhalb von 4000 atomaren Masseneinheiten (Dalton) nur die Signale der einfach geladenen (1+), zweifach geladenen (2+) und dreifach geladenen Ionen (3+) der dotiert zugegebenen Referenzsubstanz.

### Detaillierte Beschreibung

Zellen biologischen Materials wie Mikroorganismen oder Gewebezellen, die auf Probenträgerplatten aufgebracht sind, werden üblicherweise aufgeschlossen, indem eine Lösung mit Matrixsubstanz für die spätere Ionisierung durch matrixunterstützte Laserdesorption (MALDI) in einem organischen Lösungsmittel zugegeben wird. Die Matrixsubstanzen sind im Wesentlichen organische Säuren, die in Verbindung mit dem organischen Lösungsmittel die Zellwände zerstören, die Proteinkomplexe, beispielsweise die Ribosomen, im Inneren der Zellen auflösen und die Proteine aus der Zelle herauswandern lassen. Die ribosomalen Proteine eignen sich besonders für die taxonomische Identifizierung von Mikroorganismen. Beim Eintrocknen der Proben kristallisiert das Matrixmaterial aus, wobei die Proteine in die Kristalle eingebaut werden. Sie können dann im Massenspektrometer durch Laserbeschuss verdampft und durch Protonierung ionisiert werden. Falls den Zellen aber noch Nährflüssigkeit oder Teile der Aufschlussflüssigkeit anhaften, wird der Protonierungsvorgang durch Salze, andere Puffermaterialien, Detergenzien und weitere lösliche Verunreinigungen dieser Flüssigkeiten empfindlich gestört. Auch können die Zellen selbst beim Aufschluss freigesetzte Salze enthalten, die gegebenenfalls den Vorgang der Ionisierung stören.

Da Salze und andere Verunreinigungen in den Proben auf den Probenträgerplatten insbesondere eine spätere Ionisierung durch matrixunterstützte Laserdesorption (MALDI) stark stören, wird in der Erfindung vorgeschlagen, die auf dem massenspektrometrischen Probenträger befindlichen Zellen nicht einfach durch Zugabe von Matrixlösung aufzuschließen, sondern den Aufschluss der Zellen zunächst in einem eigenen Behandlungsschritt unter Verwendung von (organischen) Säuren und/oder Lösungsmitteln ohne Matrixsubstanzen vorzunehmen. Obwohl Proteine an sich nur geringe Adhäsion an metallischen Oberflächen zeigen, haften die durch diesen Aufschluss freigesetzten Proteine in verblüffender Weise recht gut auf der Oberfläche der üblichen Probenträger und können vorsichtig mit Pufferlösung (zum Beispiel wässeriger Lösung) gewaschen werden, um insbesondere Salze zu entfernen. Das funktioniert in überraschender Weise sowohl für Probenträgerplatten aus poliertem Edelstahl, wie auch für die bekannten kommerziell erhältlichen AnkerPlatten ("AnchorChip™"), die hydrophile Probenflecken ("Spots") in hydrophober Umgebung enthalten, und für die neu im Markt befindlichen "Biotargets" aus beschichtetem keramischem Material. Anscheinend werden die Proteine aus den Zellen durch die Aufschlussflüssigkeiten so weit denaturiert, dass sie genügend reaktive Adhäsionsstellen aufweisen, mit denen sie sich anheften.

Das Waschen von adhäsiv auf Oberflächen gebundenen Verdaupeptiden ist bereits bekannt, allerdings meist auf sehr speziell präparierten Oberflächen. In der Arbeit "Paraffin-waxcoated plates as matrix-assisted laser desorption/ionization sample support for high-throughput identification of proteins by peptide mass fingerprinting" (N. S. Tannu et al., Analytical Biochemistry 327 (2004) 222-232) wird das Waschen von Peptiden, die durch tryptischen Verdau von Proteinen gewonnen wurden, in ZipTip_{C18} Pipettenspitzen mit dem Waschen auf verschiedenartigen Probenträgeroberflächen verglichen, wobei das Waschen auf Ankerplatten bereits als praktikabel dargestellt wird, aber Platten mit Überzügen aus Paraffinwachs noch bessere Ergebnisse liefern sollen. Es sei hier angemerkt, dass es sich bei den interessierenden Analyten in der Arbeit von N. S. Tannu et al. um trypsin-verdaute und somit extrem stark denaturierte Peptide im Wesentlichen im Massenbereich unterhalb von m/z 3000 Dalton handelt, während die vorliegende Erfindung auf unverdaute Zell-Proteine im Massenbereich von etwa 3000 bis 20000 Dalton aus aufgeschlossenen biologische Zellen als interessierende Analyte abzielt, deren Adhäsionsverhalten mit dem von Verdauprodukten nicht verglichen werden kann. Gemäß bislang herrschender Meinung zeigen heile, nicht denaturierte Proteine nur geringe Adhäsion an Fremdoberflächen; die Einsicht, dass die schweren Proteine aus den Zellaufschlüssen an den Probenträgerplatten sogar relativ fest anhaften, kam daher vollkommen unerwartet.

In der Arbeit "Non-specific, on-probe cleanup methods for MALDI-MS samples" (Y. Xu et al., Mass-Spectrometric Reviews, 2003, 22, 429-440) werden Probenträgerplatten mit Filmen aus kommerziellen Polymeren, dünnen Schichten aus Matrix-Material, selbst-ordnenden monomolekularen Schichten und ultradünnen Polymerschichten versehen und für ihre Eignung zum Waschen darauf adsorbierter Proteine untersucht. Für einige der Oberflächen konnten Wasserstoffbrückenbildungen der Proteine nachgewiesen werden, die ein Waschen erlaubten. Es wurden keine unbeschichteten Probenträgerplatten untersucht.

Des Weiteren beschreibt die Arbeit "Compressed matrix thin film (CMTF)-assisted laser desorption ionization mass spectrometric analysis" (L. Huang et al., Analytica Chimica Acta 786 (2013) 85-94) das Waschen von Proteinen, die auf einem zu diesem Zweck zusammengepressten Dünnschichtfilm der Matrixsubstanz adhäsiv gebunden sind. Interessanterweise haben Versuche der Anmelderin mit Waschen auf Matrixdünnschichten ergeben, dass eine spätere zusätzliche Zugabe von Matrixlösung zwingend notwendig war, um aussagefähige Massenspektren zu erhalten, wenngleich die Massenspektren nur geringe Qualität aufzeigten.

Auch in der Offenlegung WO 2004/072616 (PCT/US2004/003890; J. W. Finch und J. C. Gebler; "A SAMPLE PREPARATION PLATE FOR MASS SPECTROMETRY") wird ein Waschen immobilisierter Proteine erwähnt, jedoch auf einer speziellen, für den Einsatz als Probenträger in der Massenspektrometrie ungeeigneten Präparationsplatte mit Vertiefungen für die Aufnahme der Proben und nicht auf einem massenspektrometrischen Probenträger, wie er verwendet wird, um besonders präparierte Proben für eine desorbierende Ionisierung in eine Ionenquelle einzuschieben.

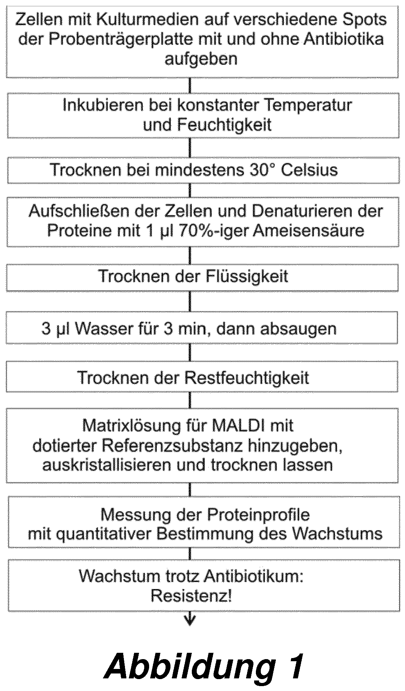

Eine erste, besonders bevorzugte Ausführungsform eines Verfahrens gemäß Prinzipien der Erfindung, das der Resistenzbestimmung von Mikroorganismen dient, kann beispielsweise die in **Abbildung 1** wiedergegeben Schritte umfassen:
(1) Die Zellen werden auf verschiedene Probenflecke einer Probenträgerplatte zusammen mit Kulturmedien (Nährlösung) ohne und mit verschiedenen Antibiotika/Antimykotika in einer oder auch verschiedenen Konzentrationen aufgegeben,
(2) es folgt eine Inkubation für eine vorgegebene Zeit in einer Kammer, die konstante Bedingungen insbesondere in Bezug auf Feuchtigkeit und Temperatur schafft, dabei kann die Zeit spezies- und antibiotika-abhängig sein,
(3) die Proben werden auf den Probenflecken bei Temperaturen zwischen 30° und 60° Celsius getrocknet,
(4) es wird 1 Mikroliter einer Lösung von 70%iger Ameisensäure in Wasser zugegeben, um die Zellen aufzuschließen und die Zell-Proteine zu denaturieren,
(5) Probenflecken bei erhöhter Temperatur trocknen lassen,
(6) 3 Mikroliter Wasser auf die Probenflecken geben, für kurze Zeit (etwa 3 Minuten) einwirken lassen und absaugen, entweder mit einer Pipette oder einer speziellen Vorrichtung zur Entfernung von Flüssigkeiten von den Probenflecken,
(7) restliche Feuchtigkeit auf den Probenflecken eintrocknen lassen,
(8) Matrixlösung (gegebenenfalls mit einem internen Standard) auf die Probenflecken pipettieren und trocknen lassen,
(9) Messung der Proteinprofile mit einem Massenspektrometer, insbesondere einem MALDI-Flugzeitmassenspektrometer,
(10) bei Wachstum trotz Antibiotikums bestimmter Konzentration liegt eine Resistenz gegenüber diesem Antibiotikum in dieser Konzentration vor; Wachstum in Nährlösungen unter Verwendung unterschiedlicher Konzentrationen des Antibiotikums erlaubt die Bestimmung der minimalen Hemmkonzentration.

Die Waschprozedur unter Verwendung deionisierten, reinen Wassers besteht in diesem Beispiel in einem Auflösen der wasserlöslichen Bestandteile in ruhendem Wasser und dem Entfernen dieser dem Ionisierungsvorgang abträglichen Bestandteile durch Entfernen des Wassers. Die Waschprozedur kann gegebenenfalls wiederholt werden. Stärkeres Waschen in bewegtem Wasser sollte vermieden werden, da es bei einigen Zell-Proteinen zu Verlusten führen kann.

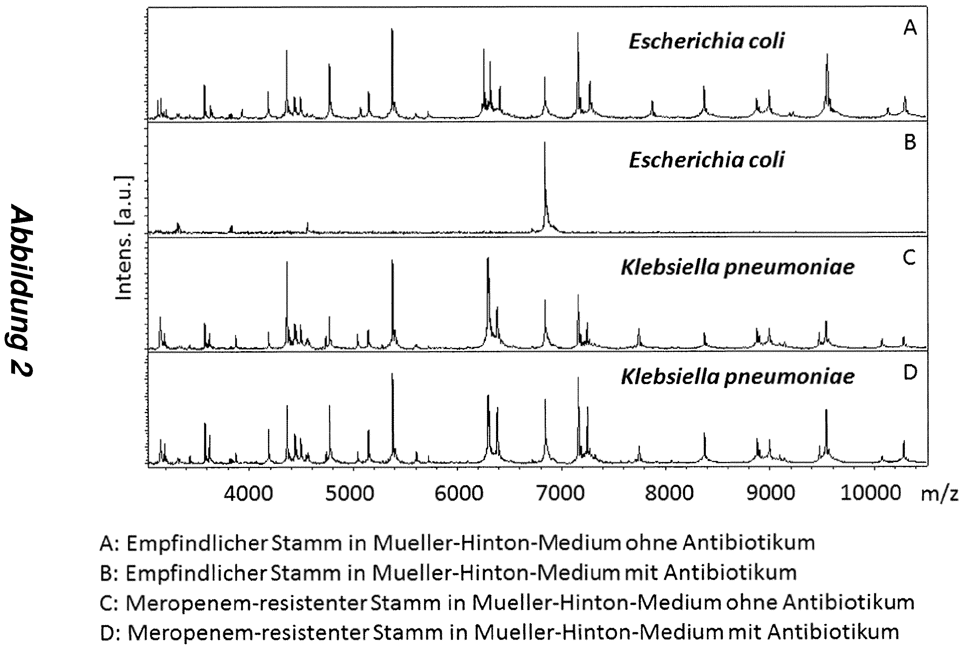

**Abbildung 2** zeigt Massenspektren eines antibiotika-empfindlichen Bakterienstammes (*E*. *coli*) und eines antibiotika-resistenten Bakterienstammes (*Klebsiella pneumoniae*), die jeweils einmal mit und einmal ohne Zugabe eines Antibiotikums gezüchtet und im Einklang mit einer Prozedur aus Abbildung 1 aufbereitet wurden. Die einzelnen Graphen illustrieren von oben nach unten:
**A**: Empfindlicher Stamm *E. coli* in Mueller-Hinton-Medium (einem Nährmedium) ohne Antibiotikum;
**B**: Empfindlicher Stamm *E. coli* in Mueller-Hinton-Medium mit Antibiotikum (hier treten nur Massensignale der dosiert zugegebenen Referenzsubstanz hervor);
**C**: Meropenem-resistenter Stamm *Klebsiella pneumoniae* in Mueller-Hinton-Medium ohne Antibiotikum; und
**D**: Meropenem-resistenter Stamm *Klebsiella pneumoniae* in Mueller-Hinton-Medium mit Antibiotikum.
Es ist an Hand der ausgeprägten Protein-Massensignale im Massenbereich über m/z 3000 in allen Spektren, in denen Wachstum nachgewiesen wird, ersichtlich, dass der Waschschritt unmittelbar auf dem Probenträger nach erfolgtem Aufschluss die aufgeschlossenen Proteine der Bakterien vom Probenträger nicht wesentlich abreichert. Das Verfahren ist demnach für die Aufbereitung von biologischen Zellproben unmittelbar auf dem Probenträger zur anschließenden desorbierenden Ionisierung und massenspektrometrischen Untersuchung, zum Beispiel zur Bestimmung der Resistenz als Zell-Eigenschaft, sehr gut geeignet.

In ähnlicher Weise können auch entartete eukaryotische Zellen, beispielsweise Krebszellen, auf Resistenz gegenüber Medikamenten getestet werden. Einzellige Pilze, ebenfalls eukaryotisch, können wie andere Mikroorganismen wenigstens bis zur taxonomischen Ebene der Art identifiziert und auf Resistenzen gegen Antibiotika (hier: Antimykotika) getestet werden. Es können aber auch mycelbildende Pilze massenspektrometrisch untersucht werden. In der Patentschrift US 8,980,577 B2 (T. Mayer, 2012) ist ein Verfahren zur taxonomischen Klassifizierung mycelbildender Pilze angegeben, das darin besteht, frische Hyphen in starker Bewegung zu kultivieren. Jedes Ansetzen an Oberflächen führt zu metabolischem Umbau, der die Massenspektren verändert. Auf diese Weise gelingt es, durch Vergleich mit Referenzspektren zu eindeutigen Identifizierungen zu kommen. Die Aufarbeitung dieser mycelbildenden Pilze aus der Flüssigkultur ist mit verschiedenen zeitaufwendigen Arbeitsschritten, wie zum Beispiel Zentrifugationen, verbunden. Nach dem hier beschriebenen Verfahren können die Pilze direkt aus der Flüssigkultur auf die Probenflecken der Probenträgerplatte aufgebracht und für die massenspektrometrische Identifizierung vorbereitet werden. Für die Prüfung auf Resistenzen gegen Antimykotika können die Pilzzellen dagegen auf den Probenflecken der Probenträgerplatten in Kulturmedien mit und ohne Antimykotika gezüchtet werden. Für die Feststellung des Wachstums in Gegenwart von Antimykotika spielt der metabolische Umbau keine entscheidende Rolle.

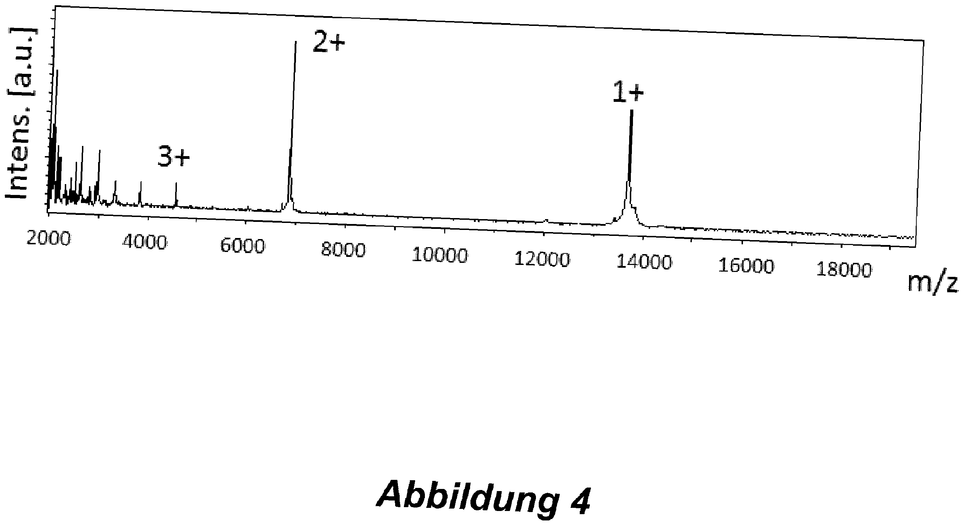

Die Nährlösung zur Kultivierung der biologischen Zellen auf dem Probenträger besteht im Allgemeinen aus Fleischextrakt und hydrolysiertem Casein, enthält also Proteine und Peptide. Die nicht entfernte, sondern nur eingetrocknete und gewaschene Nährlösung könnte somit störende Massensignale der Proteine und Peptide im Massenspektrum der Zellen erzeugen. In überraschender Weise ist das jedoch nicht der Fall, wie das Massenspektrum der **Abbildung 4** ausweist. Außer den Massensignalen (1+), (2+) und (3+) der Referenzsubstanz gibt es im verwendeten Massenbereich zwischen m/z 4000 und 18000 Dalton keinerlei störende Massensignale. Das bedeutet, dass die Peptide aus der Nährlösung entweder so klein sind, dass sie nicht stören, oder dass sie löslich bleiben und mit dem Waschschritt entfernt werden.

Das hier vorgeschlagene Verfahren mit Waschprozedur unmittelbar auf dem Probenträger dauert zwar einige Minuten länger als eines ohne, zeigt aber deutliche Vorteile: Die aus der Zelle freigesetzten Proteine bleiben während des Waschschritts erstaunlicherweise relativ fest auf der hydrophilen Oberfläche des Probenträgers haften, sie sind dadurch weitgehend unlöslich; nur die Salze und andere lösliche Bestandteile, beispielsweise aus dem Kulturmedium, gehen in Lösung und werden mit dem Waschpuffer entfernt. Das funktioniert in überraschender Weise sowohl auf Probenträgerplatten aus poliertem Edelstahl, wie auch auf den bekannten Anker-Targets ("Anchor-Chip™") und auf den neu im Markt befindlichen "Biotargets" aus keramischem Material. Folgte man hingegen der in der Patentanmeldung PCT/DE2016/100561 als bevorzugt dargestellter Vorgehensweise des Absaugens des Kulturüberstands von einem Probenfleck, so bestände die Gefahr, dass mikrobielle Spezies, die keine oder nur geringe Adhäsion zur Probenträgeroberfläche haben, verloren gehen. Als Beispiel können hier einige Salmonellenarten genannt werden, die als Flagellaten an der Flüssigkeitsoberfläche schwimmen und daher leicht abgesaugt werden könnten.

Wie Vergleichsmessungen zeigen, weisen die Spektren aus dem Verfahren, das einen Waschschritt unmittelbar auf der Probenträgeroberfläche umfasst, eine deutlich verbesserte Qualität (verbessertes Signal-zu Rausch-Verhältnis) gegenüber Spektren von ungewaschenen Proben auf, weil der Anteil an Salzen, die die Ionisierung (insbesondere beim MALDI-Verfahren) stören, durch den Waschschritt deutlich reduziert wird.

Die oben beschriebene spezielle Ausführungsform des Verfahrens kann in vielfältiger Weise abgewandelt werden. So können in Schritt (4) auch andere denaturierende flüchtige Lösungsmittel für den Aufschluss der Zellen verwendet werden, z.B. 50%iges Aceton in Wasser oder 70%ige Ameisensäure in Wasser. Es können Probenträgerplatten verwendet werden, deren Probenflecken mit Wirkstoffen vorbelegt sind, beispielsweise durch Auftrag von verschiedenartigen Antibiotika/Antimykotika oder von dem gleichen Antibiotikum/Antimykotikum in abgestuften Mengen (z.B. zur Bestimmung der minimalen Hemmkonzentration). Die Probenflecken können auch bereits dotierte Mengen an unlöslichen Referenzsubstanzen für quantitative Abschätzungen enthalten. Die Referenzsubstanzen sollen sich in der Matrixlösung auflösen können, nicht jedoch in der Pufferlösung des Waschschritts.

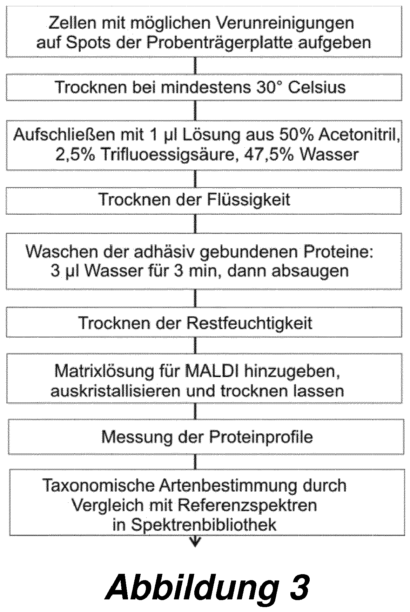

Eine andere Ausführungsform des Verfahrens gemäß Prinzipien der Erfindung ist in **Abbildung 3** wiedergeben und betrifft Mikroorganismen und andere Zellen, die nicht auf der Probenträgerplatte kultiviert werden, sondern einfach zur massenspektrometrischen Identifizierung (taxonomischen Einordnung) auf den Probenträger aufgebracht wurden, aber in der Probe Verunreinigungen wie Salze, Detergenzien und andere enthalten. Die Verunreinigungen können beispielsweise den Mikroorganismen einer Kolonie auf Agar anhaften. Bei der automatisierten Überführung von Mikroorganismen aus Kolonien auf Agar-Schalen werden häufig kleine Mengen des Agars einschließlich Nährflüssigkeit mit übertragen. Schädliche Salze können sich bei einigen Mikroorganismenarten auch in deren Inneren befinden. Diese Proben können wie bei dem oben angegebenen Verfahren ebenfalls in einem eigenen Behandlungsschritt ohne Matrixmaterial aufgeschlossen werden. Ihre denaturierten Proteine können dann ebenfalls unmittelbar auf dem Probenträger einem Waschschritt unterworfen werden.

Die desorbierende Ionisierung kann insbesondere eine Ionisierung durch matrixunterstützte Laserdesorption sein, wobei die Präparation für die Ionisierung dann das Hinzufügen und Auskristallisieren von Matrixlösung umfasst. Die Probenträgerplatte kann aus Edelstahl bestehen, eine Platte mit hydrophilen Ankern in hydrophober Umgebung ("AnchorChip™") sein, oder eine Platte aus beschichtetem Keramikmaterial.

Bei den Zellen kann es sich um Mikroorganismen handeln. Die festzustellende Eigenschaft der Mikroorganismen kann ihre Resistenz oder Suszeptibilität gegen Antibiotika/Antimykotika sein. Die Mikroorganismen können auf verschiedenen Spots der Probenträgerplatte in Nährflüssigkeit mit und ohne Zugabe von Antibiotika/Antimykotika kultiviert werden, und die Resistenz gegen ein Antibiotikum/Antimykotikum kann durch weiteres Wachstum in Anwesenheit dieses Antibiotikums/Antimykotikums bestimmt werden. Das Wachstum in Anwesenheit eines Antibiotikums/Antimykotikums kann insbesondere durch Vergleich mit einer dotiert zugegebenen Referenzsubstanz bestimmt werden.

Es können aber auch ganz allgemein biologische Zellen auf ihr Verhalten gegenüber Chemikalien während einer Kultivierung auf dem Probenträger in Gegenwart dieser Chemikalien untersucht werden. Als Beispiel kann hier das Ansprechen von Krebszellen auf verschiedene medizinische Wirkstoffe oder Wirkstoffkombinationen genannt werden.

## Patentansprüche

1. Verfahren zur Aufbereitung von Proteinen aus Proben ungereinigter biologischer Zellen auf einem Probenträger zur massenspektrometrischen Bestimmung von Zell-Eigenschaften, aufweisend die Schritte:
- Bereitstellen der biologischen Zellen auf einem Probenfleck des Probenträgers, der eine hydrophile Oberfläche oder eine Platte mit hydrophilen Ankern in hydrophober Umgebung aufweist,
- Aufschließen der Zellen unter Verwendung von denaturierenden flüchtigen Lösungsmitteln und/oder Säuren auf dem Probenfleck, wodurch sich Zell-Proteine aus Komplexen lösen, aus den aufgeschlossenen Zellen austreten und auf dem Probenfleck adhäsiv gebunden werden,
- Waschen der Zell-Proteine auf dem Probenfleck mit Pufferlösung, wobei Salze und andere lösliche Bestandteile in Lösung gehen und mit der Pufferlösung entfernt werden, und
- Präparieren der gewaschenen Zell-Proteine für eine folgende desorbierende Ionisierung.

2. Verfahren nach Anspruch 1, **dadurch gekennzeichnet, dass** der Waschschritt mit ruhender Pufferlösung vorgenommen wird.

3. Verfahren nach Anspruch 2, **dadurch gekennzeichnet, dass** für den Waschschritt einige Mikroliter einer wässerigen Lösung auf den Probenfleck aufgebracht werden, dort für eine vorbestimmte Zeitspanne verbleiben und dann abgezogen werden.

4. Verfahren nach einem der Ansprüche 1 bis 3, **dadurch gekennzeichnet, dass** die Präparation für die desorbierende Ionisierung das Hinzufügen und Auskristallisieren von Matrixlösung auf dem Probenfleck für eine spätere Ionisierung durch matrixunterstützte Laserdesorption umfasst.

5. Verfahren nach einem der Ansprüche 1 bis 4, **dadurch gekennzeichnet, dass** der eine hydrophile Oberfläche aufweisende, massenspektrometrische Probenträger eine Platte aus Edelstahl oder eine Platte aus beschichtetem Keramikmaterial aufweist.

6. Verfahren nach einem der Ansprüche 1 bis 5, **dadurch gekennzeichnet, dass** die biologischen Zellen Mikroorganismen aufweisen.

7. Verfahren nach Anspruch 6, **dadurch gekennzeichnet, dass** das Bereitstellen der biologischen Zellen die Züchtung der Mikroorganismen in Nährflüssigkeit (i) unmittelbar auf dem Probenfleck des massenspektrometrischen Probenträgers oder (ii) in einem externen Züchtungsgefäß mit anschließender Übertragung der gezüchteten Mikroorganismen auf den Probenfleck umfasst.

8. Verfahren nach Anspruch 6 oder 7, **dadurch gekennzeichnet, dass** die festzustellende Zell-Eigenschaft der Mikroorganismen ihr Resistenzverhalten gegenüber einer antimikrobiellen Substanz ist.

9. Verfahren nach Anspruch 8, **dadurch gekennzeichnet, dass** die Mikroorganismen auf verschiedenen Probenflecken des Probenträgers in Nährflüssigkeit mit und ohne Zugabe der antimikrobiellen Substanz kultiviert werden, und dass das Resistenzverhalten gegenüber der antimikrobiellen Substanz durch weiteres Wachstum in Anwesenheit dieser Substanz bestimmt wird.

10. Verfahren nach Anspruch 9, **dadurch gekennzeichnet, dass** das Wachstum in Anwesenheit der antimikrobiellen Substanz durch Vergleich mit einer dotiert zugegebenen Referenzsubstanz bestimmt wird.

11. Verfahren nach einem der Ansprüche 1 bis 10, **dadurch gekennzeichnet, dass** die biologischen Zellen auf ihr Verhalten gegenüber Chemikalien während einer Kultivierung auf dem Probenfleck des Probenträgers in Gegenwart dieser Chemikalien massenspektrometrisch untersucht werden.

12. Verfahren nach einem der Ansprüche 1 bis 11, **dadurch gekennzeichnet, dass** die Zell-Eigenschaften auf Basis von massenspektrometrischen Proteinsignalen im Massenbereich über m/z 3000 bestimmt werden.

13. Verfahren zur Aufbereitung von Proteinen aus Proben biologischer Zellen zur massenspektrometrischen Bestimmung von Zell-Eigenschaften, aufweisend die Schritte:
- Kultivieren der biologischen Zellen in Nährflüssigkeit unmittelbar auf einem Probenfleck eines massenspektrometrischen Probenträgers, der eine hydrophile Oberfläche oder eine Platte mit hydrophilen Ankern in hydrophober Umgebung aufweist, und Eintrocknen eines Kulturüberstands auf dem Probenfleck,
- Aufschließen der kultivierten Zellen auf dem Probenfleck unter Verwendung von denaturierenden flüchtigen Lösungsmitteln und/oder organischen Säuren, wodurch sich Zell-Proteine aus Komplexen lösen, aus den aufgeschlossenen Zellen austreten und auf dem Probenfleck adhäsiv gebunden werden,
- Waschen der Zell-Proteine auf dem Probenfleck mit Pufferlösung, wobei Salze und andere lösliche Bestandteile in Lösung gehen und mit der Pufferlösung entfernt werden, und
- Präparieren der gewaschenen Zell-Proteine auf dem Probenfleck für eine folgende desorbierende Ionisierung.

## Claims

1. A method for the preparation of proteins from samples of unpurified biological cells on a sample support for the mass spectrometric determination of cell properties, comprising the steps:
- providing the biological cells on a sample spot of the sample support, which has a hydrophilic surface or a plate with hydrophilic anchors in a hydrophobic environment,
- decomposing the cells using denaturing volatile solvents and/or acids on the sample spot, whereby cell proteins are released from complexes, emerge from the decomposed cells and are adhesively bound on the sample spot,
- washing the cell proteins on the sample spot with buffer solution, whereby salts and other soluble components go into solution and are removed with the buffer solution, and
- preparing the washed cell proteins for subsequent desorbing ionization.

2. The method according to claim 1, **characterized in that** the washing step is carried out with stagnant buffer solution.

3. The method according to claim 2, **characterized in that** for the washing step some microlitres of an aqueous solution are applied onto the sample spot, remain there for a predetermined period of time and are then removed.

4. The method according to any one of claims 1 to 3, **characterized in that** the preparation for desorbing ionization comprises adding and crystallizing matrix solution on the sample spot for subsequent ionization by matrix-assisted laser desorption.

5. The method according to any one of claims 1 to 4, **characterized in that** the mass spectrometric sample support having a hydrophilic surface comprises a plate of stainless steel or a plate of coated ceramic material.

6. The method according to any one of claims 1 to 5, **characterized in that** the biological cells comprise microorganisms.

7. The method according to claim 6, **characterized in that** providing the biological cells comprises culturing the microorganisms in nutrient liquid (i) directly on the sample spot of the mass spectrometric sample support or (ii) in an external culturing vessel with subsequent transfer of the cultured microorganisms onto the sample spot.

8. The method according to claim 6 or 7, **characterized in that** the cell property of the microorganisms to be determined is their resistance behaviour to an antimicrobial substance.

9. The method according to claim 8, **characterized in that** the microorganisms on different sample spots of the sample support are cultivated in nutrient liquid with and without addition of the antimicrobial substance, and that the resistance behaviour to the antimicrobial substance is determined by further growth in the presence of this substance.

10. The method according to claim 9, **characterized in that** the growth in the presence of the antimicrobial substance is determined by comparison with a doped added reference substance.

11. The method according to any one of claims 1 to 10, **characterized in that** the biological cells are mass spectrometrically examined for their behaviour towards chemicals during cultivation on the sample spot of the sample support in the presence of these chemicals.

12. The method according to any one of claims 1 to 11, **characterized in that** the cell properties are determined on the basis of mass spectrometric protein signals in the mass range above m/z 3000.

13. A method for the preparation of proteins from samples of biological cells for the mass spectrometric determination of cell properties, comprising the steps:
- culturing the biological cells in nutrient liquid directly on a sample spot of a mass spectrometric sample support which has a hydrophilic surface or a plate with hydrophilic anchors in a hydrophobic environment, and drying a culture supernatant on the sample spot,
- decomposing the cultured cells on the sample spot using denaturing volatile solvents and/or organic acids, whereby cell proteins are released from complexes, emerge from the decomposed cells and are adhesively bound on the sample spot,
- washing the cell proteins on the sample spot with buffer solution, whereby salts and other soluble components go into solution and are removed with the buffer solution, and
- preparing the washed cell proteins on the sample spot for subsequent desorbing ionization.

## Revendications

1. Procédé pour la préparation de protéines à partir d'échantillons de cellules biologiques non purifiées sur un support d'échantillon pour la détermination par spectrométrie de masse des propriétés des cellules , comprenant les étapes :
- fournir les cellules biologiques sur un point d'échantillon du support d'échantillon , qui présente une surface hydrophile ou une plaque avec des ancrages hydrophiles dans un environnement hydrophobe ,
- décomposer les cellules à l'aide de solvants et/ou d'acides volatils dénaturants sur le point d'échantillon , les protéines cellulaires étant libérées de complexes , sortant des cellules décomposées et étant liées par adhérence sur le point d'échantillon ,
- laver les protéines cellulaires sur le point d'échantillon avec une solution tampon , où les sels et autres composants solubles vont en solution et sont éliminés avec la solution tampon , et
- préparer les protéines cellulaires lavées pour une ionisation désorbante ultérieure.

2. Procédé selon la revendication 1 , **caractérisé en ce que** l'étape de lavage est effectuée avec une solution tampon stagnante.

3. Procédé selon la revendication 2 , **caractérisé en ce que** , pour l'étape de lavage , quelques microlitres d'une solution aqueuse sont appliqués sur le point d'échantillon , y restent pendant une période de temps prédéterminée et sont ensuite éliminés.

4. Procédé selon l'une quelconque des revendications 1 à 3 , **caractérisé en ce que** la préparation pour l'ionisation désorbante comprend l'addition et la cristallisation d'une solution de matrice sur le point d'échantillon pour une ionisation ultérieure par désorption laser assistée par matrice.

5. Procédé selon l'une quelconque des revendications 1 à 4, **caractérisé en ce que** le support d'échantillon pour spectrométrie de masse ayant une surface hydrophile comprend une plaque en acier inoxydable ou une plaque en matériau céramique revêtu.

6. Procédé selon l'une quelconque des revendications 1 à 5 , **caractérisé en ce que** les cellules biologiques comprennent des microorganismes.

7. Procédé selon la revendication 6 , **caractérisé en ce que** la fourniture des cellules biologiques comprend la culture des microorganismes dans un liquide nutritif (i) directement sur le point d'échantillon du support d'échantillon pour spectrométrie de masse ou (ii) dans un récipient de culture externe avec transfert ultérieur des microorganismes cultivés sur le point d'échantillon.

8. Procédé selon la revendication 6 ou 7 , **caractérisé en ce que** la propriété des cellules des microorganismes à déterminer est leur comportement de résistance à une substance antimicrobienne.

9. Procédé selon la revendication 8 , **caractérisé en ce que** les microorganismes sont cultivés sur différents points d'échantillon du support d'échantillon dans un liquide nutritif avec et sans addition de la substance antimicrobienne , et **en ce que** le comportement de résistance à la substance antimicrobienne est déterminé par une croissance continue en présence de cette substance.

10. Procédé selon la revendication 9 , **caractérisé en ce que** la croissance en présence de la substance antimicrobienne est déterminée par comparaison avec une substance de référence ajoutée dopée.

11. Procédé selon l'une quelconque des revendications 1 à 10, **caractérisé en ce que** les cellules biologiques sont examinées par spectrométrie de masse pour leur comportement vis-à-vis des produits chimiques pendant la culture sur le point d'échantillon du support d'échantillon en présence de ces produits chimiques.

12. Procédé selon l'une quelconque des revendications 1 à 11 , **caractérisé en ce que** les propriétés des cellules sont déterminées sur la base de signaux protéiques par spectrométrie de masse dans la plage de masse supérieure à m/z 3000.

13. Procédé pour la préparation de protéines à partir d'échantillons de cellules biologiques pour la détermination par spectrométrie de masse des propriétés des cellules , comprenant les étapes :
- cultiver les cellules biologiques dans un liquide nutritif directement sur un point d'échantillon d'un support d'échantillon pour spectrométrie de masse qui présente une surface hydrophile ou une plaque avec des ancrages hydrophiles dans un environnement hydrophobe , et sécher d'un surnageant de culture sur le point d'échantillon ,
- décomposer les cellules cultivées sur le point d'échantillon à l'aide de solvants et/ou d'acides organiques volatils dénaturants , les protéines cellulaires étant libérées de complexes , sortant des cellules décomposées et étant liées par adhérence sur le point d'échantillon ,
- laver les protéines cellulaires sur le point d'échantillon avec une solution tampon , où les sels et autres composants solubles vont en solution et sont éliminés avec la solution tampon , et
- préparer les protéines cellulaires lavées sur le point d'échantillon pour une ionisation désorbante ultérieure.
